## Europäisches Patentamt
## European Patent Office
## Office européen des brevets

(19)

(11) Numéro de publication: **0 088 303**
**B1**

(12) **FASCICULE DE BREVET EUROPEEN**

(45) Date de publication du fascicule du brevet:
**27.12.85**

(51) Int. Cl.⁴: **A 61 K 39/02**, A 61 K 39/095, A 61 K 39/102

(21) Numéro de dépôt: **83101843.7**

(22) Date de dépôt: **25.02.83**

(54) Procédé de préparation de complexes polysaccharide-protéine de capsules bactériennes, produits obtenus et compositions immunogènes les contenant.

(30) Priorité: **04.03.82 US 354878**

(43) Date de publication de la demande:
**14.09.83 Bulletin 83/37**

(45) Mention de la délivrance du brevet:
**27.12.85 Bulletin 85/52**

(84) Etats contractants désignés:
**AT BE CH DE FR GB IT LI LU NL SE**

(56) Documents cités:
CHEMICAL ABSTRACTS, vol. 88, no. 23, 28 février 1978, page 438, no. 168285n, Columbus, Ohio, USA, P. ANDERSON et al.: "Immunogenicity in weanling rabbits of a polyribophosphate complex from Haemophilus infuenzae type b"
CHEMICAL ABSTRACTS, vol. 86, no. 9, 28 février 1977, page 302, no. 53731w, Columbus, Ohio, USA, A. EZEPCHUK et al.: "A study of the protein-polysaccharide complex of Neisseria meningitidis serogroup A"
CHEMICAL ABSTRACTS, vol. 91, no. 5, 30 juillet 1979, page 462, no. 37312h, Columbus, Ohio, USA, W.D. ZOLLINGER et al.: "Complex of meningococcal group B polysaccharide and type 2 outer membrane protein immunogenic in man"
BIOLOGICAL ABSTRACTS, vol. 73, octobre 1982,

(73) Titulaire: **Smith Kline - RIT Société anonyme dite:, rue du Tilleul, 13, B-1320 Genval (Rixensart) (BE)**

(72) Inventeur: **Vandevelde, Jean, rue du Petit Ry, 44, B-1140 Ottignies-Louvain-la-Neuve (BE)**
Inventeur: **de Neys, Robert, rue de l'Institut, 98, B-1330 Rixensart (BE)**

(74) Mandataire: **Tasset, Gérard, SMITHKLINE - RIT rue de l'Institut, 89, B-1330 Rixensart (BE)**

(56) Documents cités: (suite)
résumé no. 68527, Philadelphia, Pennsylvenia, USA, P.R.A. ANDERSON et al.: "A polysaccharide-protein complex from Haemophilus influenzae type b: 3. Vaccine trial in human adults"

Le dossier contient des informations techniques présentées postérieurement au dépôt de la demande et ne figurant pas dans le présent fascicule.

## Description

La présente invention concerne un procédé de préparation de complexes polysaccharide-protéine de capsules bactériennes, les produits obtenus et les compositions immunogènes les contenant.

La virulence de certaines bactéries est due à la présence possible d'une capsule qui enveloppe la membrane extérieure et qui est constituée de plusieurs composants parmi lesquels des polysaccharides et des protéines.

Comme exemples de telles bactéries encapsulées, on peut citer Neisseria meningitidis, Neisseria gonorrhea, Haemophilus influenzae et Escherichia coli.

Le procédé de la présente invention et les produits ainsi obtenus ne sont pas liés à l'usage d'une souche particulière dans l'une ou l'autre espèce et la seule exigence pour le microorganisme de départ dans le procédé est d'être une bactérie encapsulée de l'espèce considérée, c'est-à-dire un matériel de départ bien connu qui peut être aisément obtenu de cas cliniques d'infection et caractérisé par l'homme de l'art.

Par exemple, les Neisseriae sont des microorganismes bien connus qui peuvent être aisément isolés de cas cliniques typiques d'infection. Ils ne sont pas doués de mouvement; ce sont des petits coques (environ 0,6 µm × 1,0 µm) à Gram négatif qui croissent isolément mais souvent en paires avec des flancs adjacents aplatis ou occasionnellement par groupes de quatre ou en grappes.

Les Neisseriae sont essentiellement aérobies mais ils se multiplient également dans des conditions microaérophiles. Parmi eux, Neisseria meningitidis groupe B est, dans une importante proportion, responsable des maladies méningococciques dans certains pays.

Dans J. Clin. Invest. 63: 836—848: 1979, W. D. Zollinger et al. rapportent que, après avoir combiné du polysaccharide partiellement purifié de Neisseria meningitidis groupe B d'une culture à des protéines de membrane extérieure préparées au départ d'une seconde culture développée dans les mêmes conditions et après avoir traité ensuite le mélange pour éliminer les lipopolysaccharides, la préparation finale était immunogène chez l'homme.

Haemophilus influenzae est également un agent pathogène à Gram négatif bien connu qui se présente sous les deux formes, encapsulée et non encapsulée. Haemophilus influenzae encapsulé contient des polysaccharides capsulaires existant sous 6 types différents (a à f), parmi lesquels le type b est un agent pathogène aux effets particulièrement graves, spécialement pour les jeunes enfants: Haemophilus influenzae type b (Hi. b), en effet, est l'une des bactéries les plus communément responsables de méningite bactérienne chez les enfants de moins de 6 ans.

Les organismes que l'on voit dans des échantillons pathologiques comme le liquide céphalo-rachidien sont généralement des petites cellules encapsulées à Gram négatif non douées de mouvement et dont la forme va de celle d'un coccobacille à celle d'un bâtonnet (0,2 µm—0,3 µm × 0,5 µm—2,0 µm). Les souches non encapsulées sont nettement pléomorphes et filamenteuses.

Sur milieu solide, les souches encapsulées virulentes apparaissent comme de petites colonies en »gouttes de rosée« et, sur milieu transparent, comme l'agar Levinthal, celles-ci sont, d'une manière caractéristique, iridescentes sous un flux de lumière oblique. Après 24—48 heures, les capsules et l'irisation disparaissent pour laisser place à une autolyse et à une réaction de coloration de Gram variable. De même, en milieu liquide, les capsules disparaissent rapidement dans les cultures.

Haemophilus influenzae est un anaérobie facultatif qui demande deux facteurs de croissance présents dans le sang: le X qui est stable à la chaleur et le V qui est labile.

Les 6 types, désignés types a à f, ont été décrits; ils sont identifiés sérologiquement par test d'agglutination, de précipitation ou de gonflement de la capsule effectué avec des antisérums spécifiques.

Dans le type b qui est, pour l'homme, le plus important au point de vue de la pathogénicité, le polysaccharide capsulaire contient du ribose, du ribitol et du phosphate et ce phosphate de polyribosylribitol est ici désigné PRR'P.

Un procédé pour la culture de Haemophilus influenzae type b et l'isolement du polysaccharide capsulaire phosphate de polyribosylribitol est décrit par L. P. Rodrigues et al. dans J. Immunol. 107: 1071—1080, 1971 et un procédé pour l'isolement et la purification du phosphate de polyribosylribitol de Haemophilus influenzae type b est donné dans le brevet No 4 220 717 des Etats-Unis d'Amérique.

Des souches de Haemophilus influenzae type b ont été déposées à l'American Type Culture Collection (ATCC) Rockville, Maryland, USA où elles ont reçu respectivement les numéros d'accès ATCC 9745 et ATCC 10211 et ces souches peuvent y être obtenues. Alternativement et comme indiqué ci-avant, des souches de Haemophilus influenzae type b peuvent également être aisément isolées de cas cliniques typiques. N'importe laquelle de ces souches et n'importe quel procédé pour la culture du microorganisme décrit dans la référence indiquée ci-avant peuvent être utilisés dans le procédé de préparation des complexes immunogènes phosphate de polyribosylribitol-protéine de la présente invention.

Le développement d'un vaccin contre Haemophilus influenzae type b pour de très jeunes enfants — c'est-à-dire en dessous de deux ans — en est encore aux premiers stades. Un vaccin à base de polysaccharide purifié a donné des résultats préliminaires encourageants mais s'est ensuite révélé induire seulement une très pauvre réponse immunitaire chez les enfants en dessous de deux ans (P. Anderson et al. J. Inf. Dis. 136: S 57—62, 1977). L'immunogénicité d'un complexe phosphate de polyribosylribitol-protéine de Haemophilus influenzae type b a également été étudié par P. Anderson et D. H. Smith (J. Inf. Dis. 136: S 63—70, 1977) sur des lapereaux récemment sevrés: dans cette étude, les auteurs indiquent que, en essayant d'analyser le fait que PRR'P est, en pratique, plus immunogène lorsqu'il est associé à la bactérie que lorsqu'il se trouve dans la forme purifiée, ils ont isolé de Haemophilus influenzae type b un complexe soluble de haut poids moléculaire dans lequel PRR'P paraît être combiné à des protéines, l'activité pyrogénique et la gélification du lysat de limule du complexe suggérant la présence d'une faible quantité de lipopolysaccharides.

Il a maintenant été trouvé que l'immunogénicité du complexe polysaccharide-protéine isolé ne dépend pas de la présence de lipopolysaccharides qui sont pyrogènes et donc des ingrédients indésirables mais dépend de la quantité et de la nature de la protéine qui y est présente. Il a également été trouvé que la liaison non covalente entre le polysaccharide et la protéine est particulièrement labile en milieu liquide en présence de produits tels que le bromure de cétrimonium et l'éthanol, lesquels sont communément employés dans la préparation de complexes de polysaccharides capsulaires bactériens.

L'invention fournit le moyen de préparer des complexes non covalents polysaccharide capsulaire bactérien-protéine immunogènes et sans lipopolysaccharide au départ de bactéries en suspension dans un milieu aqueux qui est par exemple un bouillon de culture d'un isolat pathogène naturel d'une bactérie sous forme encapsulée, ledit procédé comprenant l'inactivation de la bactérie par addition d'un sel d'ammonium quaternaire (p. ex. du bromure de cétrimonium) immédiatement suivie de la récolte de la fraction insoluble, la reprise par une solution aqueuse de 0,2 à 2 normale d'un sel de métal alcalin ou alcalino-terreux non toxique comme du chorure de sodium, du chlorure de calcium ou du chlorure de magnésium et, de préférence du chlorure de calcium, la précipitation de contaminants (principalement des acides nucléiques) par addition de 5 à 40% (vol/vol) d'un alcool miscible à l'eau (de préférence 25% d'éthanol), l'élimination du sel d'ammonium quaternaire de la solution par formation d'un sel insoluble dans l'eau, par exemple par addition d'un iodure, sulfocyanure ou benzoate (qui est de préférence un sel alcalin) soluble dans l'eau et la séparation du précipité pour donner une solution aqueuse de laquelle on isole ledit complexe, par exemple en concentrant la solution par ultrafiltration et lyophilisation.

Comme il apparaîtra des exemples, la quantité de protéine dans le complexe peut, sans porter préjudice à l'invention, varier en fonction de conditions opératoires particulières, p. ex. la concentration et/ou la nature du sel alcalin ou alcalino-terreux non toxique employé.

Pour préparer une composition immunogène convenant à l'administration à des être humains, le complexe lyophilisé est dissous dans du sérum physiologique, filtré sur gel, p. ex. de Sépharose 2 B-CL® (un produit fabriqué et vendu par Pharmacia Fine Chemicals, Uppsala, Suède) et la fraction qui n'est pas retardée est dialysée contre de l'eau, additionnée d'un stabilisant, lequel est un mono- ou di-saccharide (p. ex. du lactose). La solution est alors répartie dans des fioles de 5 ml en verre de manière à contenir 5 µg (ou un multiple de 5 µg) de complexe par dose et lyophilisée, les fioles étant ensuite bouchées hermétiquement. Avant l'administration par voie intramusculaire ou, de préférence, sous cutanée (de préférence avec un ou deux rappels), le vaccin est extemporanément reconstitué par addition de sérum physiologique apyrogène.

L'invention comprend également les compositions de valeur pour induire une immunité contre la méningite provoquée par une infection à Haemophilus influenzae type b: ces compositions comprennent un complexe immunogène polysaccharide capsulaire-protéine de Haemophilus influenzae type b non covalent et sans lipopolysaccharide et, dans ce complexe, la partie protéinique contient un résidu méthionine par molécule et, par électrophorèse sur gel de polyacrylamide-dodécylsulfate de sodium (SDS-PAGE), elle montre deux spots correspondant à ± 90 et 10% (poids/poids) de polypeptides constituant la protéine présente dans le complexe et correspondant respectivement à une sous unité hydrophobe principale présentant un poids moléculaire d'environ 40 000 et à une sous unité hydrophile en quantité moins importante et présentant un poids moléculaire d'environ 27 000, la quantité totale de protéine dans le complexe étant supérieure à 30% et, de préférence, comprise entre environ 40 et 90% du poids du complexe.

Suivant une forme préférée de l'invention, le vaccin contenant le complexe PRR'P-protéine est présenté sous forme lyophilisée additionnée d'un stabilisant (qui est constitué par un mono- ou disaccharide, comme le lactose) et le vaccin est reconstitué par addition de sérum physiologique. La préparation pharmaceutique obtenue est administrée par voie sous-cutanée ou intramusculaire.

Le complexe PRR'P-protéine de cette invention est immunogène non seulement chez l'adulte et l'enfant audessus de 6 ans mais également, comme l'indiquent les essais cliniques, chez le très jeune enfant, c'est-à-dire chez l'enfant âgé de moins de 2 ans; il ne présente aucun signe de toxicité sur hamsters au souris, il n'est pas pyrogène lorsqu'il est testé sur lapins à un dosage de 0,125 µg par millilitre par kilogramme de poids du lapin, montrant également l'absence de gélification du lysat de limule à 0,125 µg par millilitre.

Le vaccin ainsi préparé peut être employé en injektion unique ou en injections multiples pour procurer une protection suffisante contre une infection causée par Haemophilus influenzae chez l'être humain, et plus spécialement chez l'enfant âgé de moins de 2 ans. La dose unitaire unique est de 5 µg à 100 µg mais de préférence on procèdera à 2 ou 3 injections de ce genre avec intervalles de 2 à 12 semaines, par exemple 8 semaines.

L'invention est illustrée par les exemples suivants qui n'en limitent pas la portée.

Exemple 1

(a) Isolement du complexe PRR'R-protéine

Un isolat de Haemophilus influenzae type b pathogène est cultivé dans 20 l de milieu de culture liquide jusqu'au moment où la culture atteint sa phase stationnaire, comme il est décrit par P. Anderson et al. dans Infect. and Immun. 13: 581 – 9, 1976.

Les bactéries sont alors inactivées par addition de 20 g de bromure de cetrimonium et le bouillon est centrifugé pendant 7 minutes à 3000 G. Les pellets rassemblés sont repris dans 300 ml d'eau et la suspension est centrifugée pendant 7 minutes à 7000 G. Le sédiment est repris dans 400 ml d'une solution 1,8 normale de chlorure de calcium dans de l'eau et finement dispersé dans cette solution pour donner une suspension qui est agitée vigoureusement pendant une heure à 4° C. On ajoute alors 130 ml d'éthanol à 95% en maintenant encore l'agitation pendant une heure.

La suspension est centrifugée pendant 10 minutes à 20 000 G et le surnageant est dilué par addition de 1860 ml d'eau.

Le composé ammonium quaternaire est précipité par addition de 9,09 g d'iodure de potassium et le milieu est agité pendant une heure à 4° C. L'iodure de cetrimonium est alors écarté par centrifugation à 20 000 G à 4° C et le surnageant est concentré à 1/6,5 de son volume initial dans un appareil à ultrafiltration HOLLOW® (Amicon Corporation, Lexington, Mass., USA) équipé d'une cartouche à rétention de 50 000 daltons. Le rétentat est dialysé dans une membrane à dialyse VISKING® (Serva Feinbiochemica, Heidelberg, Rep. Féd. Allemande), filtré à travers 3 membranes successives (0,8, 0,45 et 0,22 µm), réparti dans des flacons en verre et lyophilisé pour donner du complexe PRR'P-protéine brut.

(b) Purification du complexe PRR'P-protéine et préparation du vaccin

Dans 100 ml d'une solution 0,4 normale de chlorure de sodium dans de l'eau, on dissout 170 mg de complexe brut et on verse la solution au sommet d'une colonne de 2 litres de gel de Sepharose 2 B-CL® (Pharmacia, Uppsala, Suède) équilibrée avec une solution 0,4 normale de chlorure de sodium dans de l'eau. La fraction qui n'est pas retardée (90,22 mg de complexe) est dialysée contre de l'eau, additionnée de 3% (poids/volume) de lactose et la solution est stérilisée par filtration à travers des membranes à 0,45 et 0,22 µ et répartie dans des flacons en verre de 5 ml contenant chacun 5 µg (ou un multiple de 5 µg) de complexe et lyophilisée. Les flacons sont ensuite bouchés hermétiquement.

Avant administration, le vaccin est reconstitué par addition de 0,5 à 5 millilitres de sérum physiologique additionné ed 0,25% de phénol par flacon.

(c) Caractéristiques physico-chimiques du complexe purifié

La fraction protéinique du complexe purifié est principalement hydrophobe. Contrastant avec les complexes précédemment décrits, l'analyse SDS-PAGE réalisée par adaptation de la méthode de Laemli (Nature 227: 680 – 685) en utilisant un gel de 3 mm d'épaisseur, une concentration de 11% (poids/volume) en gel de polyacrylamide et un tampon trométamol/glycérine à pH 8,3 pour l'élution révèle ici la présence de seulement 2 polypeptides constituant la protéine: un constituant principal (environ 90% poids/poids) constituant une fraction à 41 000 daltons et un constituant moins important (environ 10% poids/poids) constituant une fraction à 27 000 daltons. La teneur protéinique du complexe est de 54%.

La distribution du poids moléculaire à différentes concentrations dans le SDS-PAGE est indiquée dans le Tableau I et confirme la remarquable homogénéité de la fraction protéinique.

Tableau I

| Quantité déposée calculée en µg de protéine | Poids moléculaire | |
|---|---|---|
| 50 | 41.000 | 27.000 |
| 25 | 41.000 | 27.000 |
| 12,5 | 41.000 | 27.000 |
| 6,25 | 41.000 | 27.000 |
| 3,125 | 41.000 | ND |
| 1,56 | 41.000 | ND |
| 0,78 | 41.000 | ND |
| 0,39 | ND | ND |

ND: non détectable.

La protéine contient une méthionine par molécule et sa cassure au niveau méthionine par la méthode au bromure de cyanogène fournit deux fragments de, respectivement, 30 000 et 10 000 daltons.

## Exemple 2

La technique est celle de l'Exemple 1 mais on utilise au départ un échantillon de la souche Eagan (J. Anderson et al. dans J. Clin. Invest. 51: p. 31—38, 1972) à la place de l'isolat naturel qui y est indiqué et on obtient ainsi un complexe purifié présentant une teneur en protéine de 62% (poids/poids), montrant les mêmes caractéristiques que celles qui sont indiquées pour le complexe obtenu dans l'Exemple 1.

## Exemple 3

La technique est celle de l'Exemple 1 mais on utilise du chlorure de calcium 0,4 normal, du chlorure de calcium normal, du chlorure de magnésium 1,8 normal, du chlorure de sodium 0,2 normal ou du chlorure de sodium 1,8 normal à la place du chlorure de calcium 1,8 normal qui y est indiqué.

Dans ces conditions, on obtient des complexes présentant une teneur protéinique (poids/poids) de, respectivement, 34, 55, 58, 22 et 70%.

## Exemple 4

Préparation de complexe polysaccharide-protéine de Neisseria meningitidis groupe B type 2

Un isolat de Neisseria meningitidis groupe B type 2 pathogène est mis en culture dans un milieu liquide dans des conditions adaptées pour qu'une telle culture atteigne une phase de développement stationnaire, comme cela est décrit dans le brevet No 3 636 192 des Etants-Unis d'Amérique.

Une aliquote de deux litres du bouillon de culture total est inactivée par addition d'une solution aqueuse de bromure de cétrimonium à 0,1% (poids/volume) et le bouillon est centrifugé pendant 7 minutes à 7000 G.

Après inactivation, les pellets rassemblés sont conservés jusqu'au lendemain à −20°C et ensuite repris dans 40 ml d'une solution aqueuse 1,8 normale en chlorure de calcium à 4°C et la suspension obtenue est bien homogénéisée et ensuite centrifugée pendant 7 minutes à 7000 G. Le sédiment est écarté et, sous agitation énergique, on ajoute 25% (volume/volume) d'éthanol au surnageant. Après addition complète de l'éthanol, on maintient encore l'agitation pendant une heure à 4°C. Le sédiment est séparé du surnageant qui est dilué jusqu'à obtention d'une concentration finale 0,4 normale en chlorure de calcium et on refroidit le milieu dans un bain d'eau glacée.

On ajoute alors 3,66 ml d'une solution aqueuse 1,5 molaire en iodure de potassium et le milieu est agité pendant une heure à 4°C et ensuite centrifugé à 20 000 G. Le surnageant est concentré jusqu'à obtention d'un volume final de 50 ml à l'aide d'un appareil à ultrafiltration Hollow équipé d'une cartouche à rétention de 10 000 daltons et dialysé contre de l'eau à 4°C.

La solution est filtrée sur colonne Sépharose 4 B-CL® et on élue avec NaCl 0,4 normal pour obtenir une fraction non retardée qui est dialysée contre de l'eau, additionnée de 5% (poids/volume) de lactose, stérilisée par filtration et lyophilisée pour donner un complexe présentant une teneur en protéine de 60% (poids/poids).

## Exemple 5

### Préparation de complexe polysaccharide-protéine de Neisseria meningitidis groupe B type 15

Un isolat de Neisseria meningitidis groupe B type 15 est mis en culture dans un milieu liquide dans des conditions adaptées pour qu'une telle culture atteigne une phase de développement stationnaire, comme cela est décrit dans le brevet No 3 636 192 des Etats-Unis d'Amérique.

Une aliquote de trois litres de bouillon de culture total est inactivée par addition d'une solution aqueuse de bromure de cétrimonium à 0,1% (poids/volume) et le bouillon est centrifugé pendant 10 minutes à 7000 G.

Après inactivation, les pellets rassemblés sont conservés jusqu'au lendemain à −20°C et ensuite repris dans 60 ml d'une solution aqueuse 0,9 molaire en chlorure de calcium à 4°C et la suspension obtenue est bien homogénéisée. Sous agitation énergique, on ajoute 25% (volume/volume) d'éthanol et, après addition complète de l'éthanol, on maintient encore l'agitation pendant une heure à 4°C. Le sédiment est séparé du surnageant qui est dilué jusqu'à obtention d'une concentration finale 0,2 molaire en chlorure de calcium et on refroidit le milieu dans un bain d'eau glacée.

On ajoute alors 5,5 ml d'une solution aqueuse 1,5 molaire en iodure de potassium et le milieu est agité pendant une heure à 4°C et ensuite centrifugé à 20 000 G. Le surnageant est filtré sur colonne de Sephacryl S 1000® (un produit fabriqué et vendu par Pharmacia Fine Chemicals, Uppsala, Suède) et on élue avec un tampon trométamol/chlorure de calcium 0,2 molaire (pH 7) pour obtenir une fraction non retardée qui est dialysée contre de l'eau, additionnée de 3,15% (poids/volume) de lactose, stérilisée par filtration et lyophilisée pour donner un complexe présentant une teneur en protéine de 60% (poids/poids).

## Exemple 6

### Propriétés immunologiques du complexe PRR'P-protéine

#### A. Immunogénicité du complexe PRR'P-protéine chez le lapin

L'immunogénicité du complexe PRR'P-protéine a été évaluée par administration à des lapins qui, comme les enfants de moins de 2 ans, sont incapables d'élaborer une réponse anticorps contre le polysaccharide seul.

A cette fin, on a, aux jours 0, 4 et 8, injecté par voie intraveineuse à 6 lapins 3 doses successives de 10 µg de complexe PRR'P-protéine par kilo de poids. Le complexe avait été obtenu en utilisant la technique décrite ci-avant; sa teneur en protéine était de 45% (poids/poids).

Des doses de rappel ont été administrées aux jours 28, 32 et 36.

Périodiquement, on a prélevé des échantillons sanguins chez les lapins et on a déterminé les titres en anticorps bactéricides et hémagglutinants anti-PRR'P dans les sérums préalablement inactivés à 56°C pendant 30 minutes.

Les résultats sont donnés dans le Tableau II qui montre qu'une réponse anticorps maximale (en anticorps bactéricides ou anti-PRR'P) apparaît vers le jour 15, après quoi le titre en anticorps décroît rapidement.

L'administration d'une dose de rappel du complexe provoque une remotée faible et passagère du titre en anticorps hémagglutinants.

**0 088 303**

Tableau II

Immunogénicité du complexe PRR'P-protéine chez le lapin

| Jours après administration | Anticorps bactéricides (concentration exprimée en $-\log_2$) | Anticorps hémagglutinants anti-PRR'P (concentration exprimée en $-\log_2$) |
|---|---|---|
| → 0 | 3 | 0 |
| 4 | 3.2 | 1.8 |
| → 8 | 8.8 | 8.4 |
| 15 | 10.2 | 10.4 |
| 21 | 7.5 | 4.5 |
| →28 | 7.0 | 4.5 |
| →32 | 6.0 | 6.9 |
| →36 | 4.0 | 5.9 |
| 44 | 5.0 | 5.0 |
| 59 | 4.0 | 5.0 |
| 57 | 4.0 | 4.0 |
| 63 | 2.0 | 4.0 |
| 78 | — | 3.0 |

Comme contrôle, on a administré à deux lapins la même dose de PRR'P seul (préparé en suivant la technique décrite par E. C. Gotschlich pour les polysaccharides des groupes A et C dans le brevet No 3 636 192 des Etats-Unis d'Amérique), en suivant le même programme pour l'administration, les prises de sang et la détermination du titre en anticorps et on n'a détecté aucune réponse.

Pour déterminer la relation entre la teneur en protéine et l'immunogénicité du complexe, on a administré à intervalles de 4 jours et par voie intraveineuse à 5 groupes de 3 lapins, 3 doses de complexe PRR'P-protéine contenant chacune 2,5 µg de PRR'P par kilo de poids, les pourcentages (poids/poids) en protéine dans le complexe étant respectivement 11, 30, 48, 69 et 86.

Un sixième groupe de 3 lapins a été utilisé comme contrôle suivant le même programme mais recevant les mêmes doses de PRR'P (2,5 µg) en lieu et place de complexe PRR'P-protéine.

On a prélevé des échantillons sanguins chez les lapins aux mêmes intervalles de temps et les titres en anticorps anti-PRR'P déterminés par technique ELISA sont donnés dans le Tableau III.

7

Tableau III

Relation entre immunogénicité et teneur en protéine du complexe PRR'P-protéine

| Jours après la première administration | Titre en anticorps anti-PRR'P (en unités ELISA) Pourcentage (poids/poids) de protéine dans le complexe | | | | | |
| --- | --- | --- | --- | --- | --- | --- |
| | 0 | 11 | 30 | 48 | 60 | 86 |
| → 0 | 0 | 0 | 0 | 0 | 0 | 0 |
| → 4 | 2.7 | 2.8 | 3.0 | 1.6 | 1.2 | 3.6 |
| → 8 | 10 | 19.2 | 19.2 | 36.7 | 25.7 | 13.0 |
| 12 | 15.8 | 20.0 | 28.2 | 117.5 | 96.7 | 87.5 |
| 15 | 16.7 | 37.5 | 26.7 | 141.7 | 130.0 | 145.0 |
| 19 | 25.8 | 21.7 | 27.5 | 69.2 | 68.3 | 112.0 |
| 22 | 25.8 | 31.7 | 39.2 | 59.2 | 53.3 | 42.5 |
| 25 | 63.3 | 36.7 | 31.7 | 53.3 | 48.3 | 55.0 |

Du Tableau III il appert que, après l'administration de PRR'P seul ou d'un complexe contenant au maximum 30% de protéine, il y a seulement un faible accroissement du titre en anticorps anti-PRR'P. Au contraire, les complexes qui contiennent au moins 48% de protéine sont fortement immunogènes et on n'observe pas de différence substantielle dans l'immunogénicité pour les complexes qui présentent une teneur en protéine comprise entre 48 et 95%.

Pour élucider quelle classe ou quelles classes d'anticorps est ou sont impliquée(s) dans la réponse au complexe, on a immunisé 5 lapins avec du complexe PRR'P-protéine (obtenu en utilisant la technique de l'Exemple 1 et présentant une teneur (poids/poids: en protéine de 45%) administré par voie intraveineuse en 3 doses successives de 10 µg par kilo de poids aux jours 0, 4 et 8 et on a administré une même dose de rappel par voie intraveineuse aux jours 43 et 46. Les titres en anticorps anti-PRR'P ont alors été déterminés par hémagglutination à différents moments simultanément en présence ou en absence de 2-mercaptoéthanol 0,1 molaire (c'est-à-dire un réactif bien connu pour dissocier les IgM en leurs monomères et laissant les IgG intacts). Les résultats obtenus avec un lapin sont donnés dans le Tableau IV suivant; les profils de réponse chez les autres lapins montrent des résultats comparables différant principalement en ce qui concerne l'intensité de la réponse.

Tableau IV

Effet du 2-mercaptoéthanol (2-ME) sur le titre en anticorps hémagglutinants anti-PRR'P

| Jours après la première administration | Titre en anticorps hémagglutinants anti-PRR'P | |
|---|---|---|
| | sans 2-ME | avec 2-ME |
| 0 | 0 | 0 |
| 3 | 0 | 1 |
| 8 | 5 | 1 |
| 15 | 8 | 1 |
| 20 | 10 | 1 |
| 43 | 7 | 1 |
| 46 | 6 | 0 |
| 50 | 12 | 4 |
| 58 | 10 | 3 |
| 64 | 7 | 1 |
| 71 | 6 | 1 |

Les valeurs du Tableau IV montrent que la plus grande partie du titre hémagglutinant observé après les 3 premières injections est due aux IgM puisque les propriétés d'hémagglutination sont en grande partie disparues après traitement au mercaptoéthanol. Néanmoins, une faible hémagglutination résiduelle indique que de petites quantités de IgG sont présentes dès le jour 3.

Après les dernières injections, on observe un effet de rappel avec ou sans traitement au mercaptoéthanol, ce qui suggère la production à la fois de IgG et de IgM après la seconde série d'injections. Cependant, la réponse IgG est plus forte après l'administration de rappel qu'après les injections initiales.

En conséquence, les figures du Tableau IV démontrent que, à la fois IgG et des IgM sont produites après immunisation avec le complexe PRR'P-protéine, ce qui suggère que le complexe PRR'P-protéine est un antigène thymodépendant.

La thymodépendance est confirmée par le profil de réponse obtenu lorsqu'on administre le complexe PRR'P-protéine par voie intraveineuse au jour 0 et 2 semaines plus tard.

Le Tableau V qui suit donne les titres en anticorps anti-PRR'P (exprimés en unités ELISA) obtenus chez des lapins à qui, aux jours 0 et 15, on a injecté, par voie intraveineuse, 30 µg de complexe PRR'P-protéine présentant une teneur de 45% (poids/poids) en protéine.

Tableau V

Cinétique de la réponse anticorps anti-PRR'P après immunisation avec le complexe
PRR'P-protéine

| Jours après la première administration | Titres en anticorps anti-PRR'P (unités ELISA) | | |
|---|---|---|---|
| | lapin 1 | lapin 2 | lapin 3 |
| → 0 | 32 | 28 | 40 |
| 4 | 60 | 41 | 60 |
| 9 | 52 | 55 | 57 |
| 14 | 36 | 35 | 32 |
| →16 | 48 | 36 | 43 |
| 18 | 112 | 74 | 104 |
| 22 | 161 | 149 | 35 |
| 28 | 40 | 46 | 34 |

### B. Stimulation des cellules de la rate de lapin in vitro (transformation lymphoblastique)

On a immunisé trois lapins avec soit le complexe PRR'P-protéine (à 45% (poids/poids) en protéine) soit avec le polysaccharide seul (3 injections successives de 10 μg de complexe PRR'P-protéine ou de PRR'P par kilo de poids aux jours 0, 4 et 8, suivant le programme indiqué ci-avant). On a prélevé la rate de chaque lapin au jour 15, lequel correspond au titre le plus élevé en anticorps sériques chez les lapins qui reçoivent du complexe PRR'P-protéine. Les cellules de rate ont alors été mises en culture pendant 5 jours (à 37°C en atmosphère humidifiée contenant 5 de $CO_2$ et à une densité cellulaire de $3.10^6$ celluless par millilitre) en utilisant du milieu RPMI 1640 (comme décrit par G. E. Moore et al. dans J. A. M. A. 199: 519—524; 1967) et additionné, de L-glutamine, 2 millimolaire; pénicilline G, 100 UI/ml; 2-mercaptoéthanol, $5.10^{-5}$ molaire et 10% de sérum normal de lapin en présence de complexe PRR'P-protéine ou de polysaccharide seul, à différentes concentrations allant de 1 à 100 μg/ml. A la fin de la période d'incubation, on a ajouté aux cultures 5 μCi de 3 thymidine et les cellules ont été récoltées et lavées 18 heures plus tard à l'aide d'un récolteur automatique MASH II (Flow Laboratories, Inc., Rockville, Maryland, USA).

La radioactivité incorporée dans les cellules a été mesurée à l'aide d'un compteur liquide Packard Tri-Carb® (Packard Instr. Co., DOWNERS Grove, Illinois, USA).

Les résultats sont présentés au Tableau VI.

Tableau VI

Stimulation in vitro de lymphocytes de rate de lapins par le PRR'P et par le complexe
PRR'P-protéine

| Concentr. en antigènes dans la culture (ug/ml) | Coups par minute incorporés par culture ($\times 10^{-3}$) | | | |
| --- | --- | --- | --- | --- |
| | Lapins injectés avec PRR'P Stimulation par | | Lapins injectés avec le complexe PRR'P-protéine Stimulation par | |
| | PRR'P | complexe | PRR'P | complexe |
| 0 | 66 ± 34 | 66 ± 34 | 512 ± 221 | 512 ± 221 |
| 1 | 1157 ± 447 | 1025 ± 760 | 1419 ± 1392 | 5 211 + 1713 |
| 2.5 | 877 ± 293 | 2079 ± 1261 | 2985 ± 664 | 9 276 ± 1056 |
| 5 | 830 ± 340 | 2202 ± 409 | 1916 ± 389 | 17 116 ± 3232 |
| 10 | 862 ± 234 | 815 ± 318 | 3082 ± 344 | 25 307 ± 5749 |
| 25 | 711 ± 360 | 624 ± 236 | — | 53 369 ± 1108 |
| 50 | 717 ± 281 | 479 ± 147 | 3210 ± 580 | 77 031 ± 3549 |
| 100 | 1481 ± 171 | 602 ± 306 | 651 ± 170 | 88 544 ± 5463 |

Le Tableau VI montre que les cellules de rate des lapins immunisés avec du PRR'P ne répondent ni au complexe ni au polysaccharide. Cette constatation est en accord avec l'absence d'anticorps anti-PRR'P dans le sérum des lapins immunisés avec le seul polysaccharide. D'autre part, les cellules de rate des lapins immunisés avec le complexe PRR'P-protéine incorporent de grandes quantités de $^3$H-thymidine montrant un taux élevé de prolifération en présence de complexe PRR'P-protéine, la prolifération étant an relation avec la concentration en antigènes. Les cellules de rate ne prolifèrent pas lorsque, au lieu d'ajouter du complexe PRR'P-protéine, on ajoute seulement du PRR'P.

De cet essai, on peut conclure que, contrairement au PRR'P seul, le complexe PRR'P-protéine peut induire la prolifération d'une population de cellules T dans la rate des lapins immunisés avec le complexe PRR'P-protéine. Cette population de cellules T peut être stimulée in vitro par le complexe PRR'P-protéine mais ne peut pats être stimulée par le PRR'P seul, ce qui suggère que le déterminant reconnu par les cellules T est présent dans la fraction protéinique du complexe.

Le test de transformation lymphoblastique a également été utilisé pour détecter la présence de cellules-mémoire dans la rate des lapins, quelques mois après leur immunisation avec du complexe PRR'P-protéine.

Dans ce but, on a immunisé un lapin avec du complexe PRR'P-protéine (45% (poids/poids) en protéine) suivant le programme indiqué ci-avant (par 3 injections intraveineuses de 10 µg de complexe PRR'P-protéine par kilo de poids avec des doses de rappel aux jours 43 et 46). L'administration de rappel a induit une rapide élévation du titre en anticorps anti-PRR'P mais, comme il fallait s'y attendre, ce titre est tombé rapidement au cours de la quinzaine suivante.

Vingt jours après le dernier rappel, le titre en anticorps était moins d'un quart de la valeur atteinte au jours 50.

Les résultats sont indiqués dans le Tableau VII A suivant.

Tableau VII A

Persistance de cellules-mémoire dans la rate: Cinétique de la réponse anticorps

| Jour | Titre en anticorps anti-PRR'P (unités ELISA) dans le sérum |
|------|-----|
| → 0 | 5.8 |
| → 3 | 4.1 |
| → 8 | 19.0 |
| 15 | 28.0 |
| 20 | 36.0 |
| →43 | 19.0 |
| →46 | 4.0 |
| 50 | 200.0 |
| 58 | 80.0 |
| 64 | 44.0 |
| 71 | 40.0 |

Un abaissement ultérieur du titre en anticorps a été montré expérimentalement 100 jours après l'immunisation initiale au moment du prélèvement de la rate.

Les cellules ($6.10^5$) isolées de la rate ont été mises en culture pendant 5 jours à 37°C en atmosphère humidifiée contenant 5% de $CO_2$ en utilisant du milieu RPMI 1640 additionné de 10% de sérum de lapin normal autologue et inactivé à la chaleur et contenant différentes concentrations (jusqu'à 100 μg/ml) de PRR'P ou de complexe PRR'P-protéine (teneur (poids/poids) en protéine: 45%). Le cinquième jour, les cellules ont été mises en contact pendant 18 heures avec 1 μCi de 6 $^3$H-thymidine et on a mesuré la radioactivité incorporée dans les cellules.

Les résultats sont donnés au Tableau VI B et montrent que les cellules de rate prolifèrent en réponse au complexe PRR'P-protéine mais ne prolifèrent pas en réponse au PRR'P seul.

On peut conclure que des cellules-mémoire (cellules T) persistent dans la rate des lapins immunisés 3 mois et demi auparavant et à qui on a administré des doses de rappel 1 mois et demi après la première immunisation, à un moment où on ne détectait plus d'anticorps dans le sérum.

Tableau VII B

Persistance de cellules-mémoire dans la rate: Prise de $^3$H-thymidine par les cellules de rate

| Concentration en antigènes (μg/ml) dans la culture | Coups incorporés par minute par 6.10$^5$ cellules de rate ($\times 10^{-3}$) de lapins ayant reçu le complexe PRR'P-protéine |
|---|---|
| 0 | 792 |
| 1 | 915 |
| 2.5 | 976 |
| 5 | 833 |
| 10  PRR'P | 1380 |
| 25 | 939 |
| 50 | 831 |
| 100 | 1113 |
| 0 | 690 |
| 1 | 858 |
| 2.5 | 2432 |
| 5 | 4592 |
| 10  Complexe | 6122 |
| 25 | 7957 |
| 50 | 7786 |
| 100 | 2422 |

La capacité d'élaboration d'une réponse anticorps secondaire manifestée par les cellules de rate prélevées au jour 100 (comme il est indiqué ci-avant) a été évaluée comme suit:

Les cellules de rate ont été mises en culture pendant 6 jours à 37°C en atmosphère humidifiée contenant 5% de $CO_2$, en utilisant des tubes plastiques à fond rond à une concentration de 3.10$^6$ cellules/ml dans un millilitre de milieu RPMI 1640 additionné de glutamine 2 millimolaire, de mercaptoéthanol 5.10$^{-5}$ molaire, de 100 UI/ml de pénicilline G, de 100 μg/ml de steptomycine et de 10% de sérum de lapin normal inactivé à la chaleur. L'antigène (PRR'P ou complexe) a été ajouté aux cultures à différentes concentrations jusqu'à 25 μg/ml.

Après 6 jours, les cellules ont été lavées par centrifugation avec du milieu RPMI pour éliminer l'antigène et mises en culture pendant un jour en utilisant le même milieu mais sans antigène et pendant un autre jour en utilisant le milieu additionné de 10% de sérum foetal de veau pour éliminer les protéines de sérum de lapin. Les cellules ont alors été lavées et cultivées pendant 3 autres jours dans le milieu contenant du sérum foetal de veau. A la fin de cette période de culture, les cellules ont été sédimentées et le surnageant recueilli par détermination des anticorps anti-PRR'P par la technique ELISA, en utilisant des IgG de chèvre anti IgG de lapin et marqués à la peroxydase.

Les résultats sont donnés dans le Tableau VII C suivant; ils indiquent clairement que les cellules de rate sont capables de produire des anticorps anti-PRR'P in vitro lorsqu'ils sont incubés avec le complexe PRR'P-protéine; cette réponse est dépendante de la dose et on ne constats aucune production d'anticorps anti-PRR'P en réponse au polysaccharide.

Tableau VII C

Persistance des cellules-mémoire dans la rate:
Production d'anticorps anti-PRR'P par les cellules de rate in vitro.

| Concentration en antigènes (μg/ml) dans la culture | Titre en anticorps anti-PRR'P (densité optique à 450 nm) |
|---|---|
| 0 | 0.141 |
| 1 | 0.159 |
| 2.5 | 0.161 |
| 5   PRR'P | 0.168 |
| 10 | 0.161 |
| 25 | 0.169 |
| 0 | 0.130 |
| 1 | 0.353 |
| 2.5 | 0.563 |
| 5   Complexe | 0.575 |
| 10 | 0.325 |
| 25 | 0.310 |

## Exemple 7

### I. Test cliniques chez les adolescents

Un groupe de 82 adolescents en bonne santé, masculins et féminins (âgés de 15 à 16 ans) a été divisé en 2 sous-groupes de 41.

Avant vaccination, on a prélevé des échantillons de sérum pour la détermination des anticorps et on a administré par voie souscutanée au premier sous-groupe du vaccin PRR'P-protéine obtenu suivant l'Exemple 1 tandis qu'on administrait de la même manière du vaccin PRR'P au second sous-groupe.

Le dosage était de 21 μg de complexe PRR'P protéine pour le premier sous-groupe et de 13,7 μg de PRR'P pour le second sous-groupe avec une administration de rappel pour chaque sous-groupe, 4 semaines après la première injection.

Les tests ont été effectués en conditions »double aveugle«.

Parmi les sujets qui avaient reçu le vaccin complexe PRR'P-protéine et après la première administration, 2 sujets ont fait état d'un léger érythème local et un autre a présenté un léger mal de tête et, après l'administration de rappel, 2 sujets ont signalé de légers symptômes généraux et locaux et un sujet a manifesté une réaction locale.

Parmi les sujets qui avaient reçu le vaccin PRR'P et après la première administration, un subjet a montré une légère réaction locale et générale et, après l'administration du rappel, le même sujet a montré un léger endolorissement local.

Les résultats sérologiques obtenus par ELISA ont montré une augmentation d'au moins deux fois du titre en anticorps chez 71% des sujets qui avaient reçu le vaccin complexe de PRR'P-protéine et chez 50% des sujets qui avaient reçu le vaccin PRR'P.

Une augmentation de 4 fois dans le titre en anticorps bactéricides a été obtenue respectivement chez 75% et 63% des sujets.

**0 088 303**

## II. Test cliniques chez les enfants

Un groupe de 71 enfants masculins et féminins (tous âgés de 6 ans) a été divisé en 2 sous-groupes comprenant respectivement 32 et 39 enfants.

Avant vaccination, des échantillons de sérum avaient été prélevés pour la détermination des anticorps et on a administré par voie souscutanée au premier sous-groupe de vaccin complexe PRR'P-protéine obtenu suivant l'Exemple 1 et, au second sous-groupe, du vaccin PRR'P.

Le dosage unitaire était de 10 µg de complexe pour le premier sous-groupe et de 6,35 µg de PRR'P pour le second sous-groupe avec, pour chaque sous-groupe, une administration de rappel, 4 semaines après la première injection.

Les tests ont été effectués en conditions »double aveugle«.

Parmi les enfants qui avaient reçu le vaccin complexe PRR'P-protéine et après la première administration, 2 enfants ont signalé un léger érythème local et, après l'administration de rappel, 2 enfants ont signalé un léger endolorissement.

Parmi les enfants qui avaient reçu le vaccin PRR'P et après la première administration, un enfant a manifesté un léger endolorissement, un autre a eu un mal de tête et un troisième a présenté de la fièvre et, après l'administration de rappel, 3 ont fait état d'un léger endolorissement local.

Les résultats sérologiques obtenus par ELISA ont montré une augmentation du titre en anticorps anti PRR'P d'au moins deux fois chez 68,8% des enfants après la première injection de vaccin complexe PRR'P-protéine et chez 62,5% après l'administration de rappel du même vaccin et, chez les enfants qui avaient reçu le vaccin PRR'P, les résultats ont été respectivement de 71,9% et de 76,9%.

Pour les anticorps bactéricides, les résultats étaient respectivement de 66% et 75% pour le premier sous-groupe et de 79% et 82% pour le second sous-groupe.

## III. Test cliniques chez les nourrissons

Par voie souscutanée, on a administré du vaccin complexe PRR'P-protéine obtenu suivant l'Exemple 1 à 4 nourrissons (âgés de 4 à 8 mois) en suivant le programme de vaccination décrit plus haut, le dosage unitaire étant de 13,5 µg par nourrisson.

Les taux d'anticorps (IgG, IgA et IgM) ont été déterminés par ELISA avant et après administration du vaccin, à l'aide d'un standard humain contenant 32 µg d'anticorps précipitants anti-PRR'P par millilitre. Les résultats sont résumés dans le Tableau VIII dans lequel les valeurs entre parenthèses donnent l'augmentation de titre après la première administration (Post-1) et après l'administration de rappel (Post-2).

Les chiffres montrent que, après la première administration, on observe une augmentation de 2 fois du titre chez 2 enfants pour IgG et chez un enfant pour IgA ou IgM. Après l'administration de rappel, une augmentation du titre de deux fois a été obtenue chez 4 enfants pour IgG, chez 2 enfants pour IgA et chez un enfant pour IgM.

Tableau VIII

Réponse en anticorps anti-PRR'P après immunisation de nourrissons avec le vaccin PRR'P-protéine

| Nourrisson No | IgG (ng/ml) | | | | IgA (ng/ml) | | | | IgM (ng/ml) | | | |
|---|---|---|---|---|---|---|---|---|---|---|---|---|
| No | 1 | 3 | 4 | 5 | 1 | 3 | 4 | 5 | 1 | 3 | 4 | 5 |
| Age (mois) | 4 | 8 | 4 | 4.5 | 4 | 8 | 4 | 4.5 | 4 | 8 | 4 | 4.5 |
| Titre en anticorps avant vaccination | 160 | 520 | 360 | 240 | 0 | 880 | 320 | 480 | 350 | 1500 | 600 | 400 |
| Post-1 | 400 | 880 | 560 | 640 | 440 | 960 | 400 | 560 | 1000 | 1400 | 850 | 700 |
| | (2.5) | (1.7) | (1.6) | (2.7) | (> 44) | (1.1) | (1.3) | (1.2) | (2.9) | (0.9) | (1.4) | (1.8) |
| Post-2 | 1320 | 1480 | 2560 | 520 | 400 | 480 | 5760 | 400 | 3400 | 2100 | 2950 | 1600 |
| | (8.3) | (2.8) | (7.1) | (2.2) | (> 50) | (0.6) | (18.0) | (0.8) | (9.7) | (1.4) | (4.9) | (4.0) |

15

De ces résultats, il appert que le vaccin complexe PRR'P-protéine de l'invention et le vaccin PRR'P sont équivalemment valables pour immuniser des adultes et des enfants sans manifester d'effets secondaires substantiels. De plus, le vaccin complexe PRR'P-protéine est également efficace chez les jeunes enfants (endessous de 18 mois).

## Exemple 8

### Réponse anticorps au complexe polysaccharide de Neisseria meningitidis groupe B-protéine chez le lapin

A 3 lapins, on a injecté par voie intraveineuse du complexe protéine-polysaccharide de Neisseria meningitidis groupe B Type 2 tel qu'obtenu à l'Exemple 4, aux jours 0, 4 et 8 et à la dose de 10 µg de complexe par kilo de poids d'animal. Comme contrôle, on a injecté par la même voie à un second groupe de 3 lapins et aux jours 0, 4 et 8 du polysaccharide de Neisseria meningitidis groupe B Type 2 (PSB) à la dose de 5 µg par kilo de poids. On a prélevé des échantillons de sang chez les lapins des 2 groupes à intervalles de 4 jours et, par ELISA, on a déterminé les titres en anticorps anti-PSB dans les différents sérums. Les taux en anticorps bactéricides ont été déterminés dans les mêmes sérums en utilisant soit la souche de laquelle était dérivé le complexe protéine-polysaccharide (souche contenant l'antigène sérotype 2 ou STA 2) ou en utilisant une souche du groupe B mais de sérotype différent (contenant l'antigène sérotype 6 ou STA 6). Le dernier test permet de faire la distinction entre les anticorps bactéricides PSB et les anticorps de différentes spécificités. Les résultats sont résumés dans le Tableau IX. Ils montrent clairement que, contrairement au polysaccharide seul, le complexe est capable d'éliciter une réponse anticorps anti-PSB. Le taux en anticorps est maximal entre les jours 8 et 12 et décroit lentement par la suite pour devenir non significatif 30 jours après la première administration. L'immunisation avec le complexe élicite également des anticorps bactéricides. Certains sont bactéricides pour la souche contenant STA 6 qui n'a que PSB en commun avec la souche STA 2 et montre la même cinétique que les anticorps anti-PSB.

Ces résultats démontrent que les anticorps anti-PSB induits par le complexe chez le lapin sont bactéricides. La présence, au jour 30, d'une concentration significative en anticorps bactéricides pour la souche contenant STA 2 mais pas pour la souche contenant STA 6 indique, de plus, que certains anticorps bactéricides sont dirigés directement contre le composant protéinique du complexe.

Pour investiguer si les anticorps bactéricides présents au jour 30 sont dirigés contre l'antigène du sérotype ou contre un autre antigène somatique, on a utilisé le sérum d'un lapin immunisé comme indiqué ciavant avec un complexe protéine-polysaccharide obtenu par la méthode de l'Exemple 4 mais contenant 79% de protéine pour tester sa teneur en anticorps bactéricides contre plusieurs souches de N. meningitidis B contenant des antigènes de différents sérotypes. Le sérum obtenu 29 jours après la première administration de complexe ne contenait pas d'anticorps anti-PSB, d'après la méthode ELISA. Ce sérum était bactéricide pour la souche employée pour préparer le complexe (contenant STA 2) et pour plusieurs souches de N. meningitidis groupe B ou groupe C mais contenant STA 2.

Cependant, le résum était incapable de tuer des bactéries ayant un antigène d'un autre sérotype (c'est-à-dire respectivement STA 1, 4, 6, 8, 9, 11, 12 et 14). C'est pourquoi il faut conclure que l'antigène du sérotype 2 (STA 2) est au moins un des composants de la fraction protéinique du complexe décrit dans l'exemple 4.

Tableau IX

Réponse anticorps chez les lapins immunisés avec le complexe PSB-protéine ou avec PSB seul

| Jours après la première administration | Anticorps(*) ELISA anti-PSB (densité optique à 450 nm) complexe | PSB | Anticorps(*) bactéricides induits par le complexe (-log$_2$) Souche contenant STA 2 | STA 6 |
|---|---|---|---|---|
| → 0 | 0.029 | 0.017 | 0 | 1.3 |
| → 4 | 0.028 | 0.028 | 3.7 | — |
| → 8 | 0.250 | 0.028 | 8 | 8 |
| 12 | 0.241 | 0.036 | 7 | — |
| 15 | 0.147 | 0.023 | 5.7 | 4 |
| 30 | 0.045 | 0.043 | 2.7 | 1.3 |

(*)   Ces valeurs sont soit la moyenne géométrique (anticorps ELISA) soit la moyenne arithmétique (anticorps bactéricides) des valeurs obtenues pour les 3 lapins.

## Revendications pour les etats contractants: BE, CH, DE, FR, GB, IT, LI, LU, NL, SE

1. Procédé de préparation d'un complexe capsulaire bactérien polysaccharide-protéine non covalent, immunogène et sans lipopolysaccharides au départ de bactéries encapsulées en suspension dans un milieu aqueux, comprenant l'inactivation des bactéries par addition d'un sel d'ammonium quaternaire suivie de la récolte de la fraction insoluble, de sa reprise dans une solution aqueuse de 0,2 à 2 normale en sel de métal alcalin ou alcalino-terreux non toxique, de la précipitation des contaminants autres que le sel d'ammonium quaternaire par addition de 5 à 40% (volume/volume) d'un alcool miscible à l'eau suivie de l'élimination du sel d'ammonium quaternaire, caractérisé en ce qu'on élimine le sel d'ammonium quaternaire par précipitation par ajout d'un iodure, sulfocyanure ou benzoate soluble dans l'eau et qu'on sépare le précipité pour obtenir une solution de laquelle on isole ledit complexe.

2. Procédé suivant la revendication 1, dans laquelle la bactérie est Haemophilus influenzae type B.

3. Procédé suivant la revendication 1, dans laquelle la bactérie est Neisseria meningitidis groupe B.

4. Procédé suivant l'une quelconque des revendications 1 à 3, dans laquelle le milieu aqueux est un bouillon de culture.

5. Procédé suivant l'une quelconque des revendications 1 à 4, dans laquelle le sel d'ammonium quaternaire est le bromure de cétrimonium.

6. Procédé suivant l'une quelconque des revendications 1 à 5, dans laquelle le sel de métal non toxique est le chlorure de calcium.

7. Procédé suivant l'une quelconque des revendications 1 à 6, dans laquelle la quantité d'alcool miscible à l'eau est 25% (volume/volume) et l'alcool est l'éthanol.

8. Procédé suivant l'une quelconque des revendications 1 à 7 dans laquelle l'iodure soluble dans l'eau est un iodure de métal alcalin.

9. Procédé suivant l'une quelconque des revendications 1 à 8, dans laquelle l'isolement du complexe de la solution aqueuse est réalisé par ultrafiltration et lyophilisation.

10. Complexe capsulaire polysaccharide-protéine non covalent de Haemophilus influenzae type b, immunogène et sans lipopolysaccharide caractérisé en ce que la fraction polysaccharidique est constituée par du phosphate de polyribosylribitol et la fraction protéinique présente un reste méthionine par molécule et que, lorsqu'il est analysé par électrophorèse sur gel de polyacrylamide — dodécyle sulfate de sodium, il montre deux spots correspondant resprectivement à environ 90% et 10% (poids/poids) de polypeptides constituant la protéine présente dans le complexe, la fraction principale étant hydrophobe et présentant un poids moléculaire d'environ 41 000 daltons, la teneur totale en protéine dans le complexe étant d'au moins 30% poids du complexe.

11. Complexe suivant la revendication 10 dans laquelle la teneur en protéine est comprise entre 40 et 90% (poids/poids) du complexe.

12. Vaccin contre la méningite caractérisé en ce qu'il comprend une dose efficace du complexe polysaccharide-protéine suivant l'une quelconque des revendications 10 et 11 sous forme lyophilisée, avec un stabilisant.

17

**0 088 303**

## Revendications pour l'etat contractant: AT

1. Procédé de préparation d'un complexe capsulaire bactérien polysaccharide-protéine non covalent, immunogène et sans lipopolysaccharides au départ de bactéries encapsulées en suspension dans un milieu aqueux, comprenant l'inactivation des bactéries par addition d'un sel d'ammonium quaternaire suivie de la récolte de la fraction insoluble, de sa reprise dans une solution aqueuse de 0,2 à 2 normale en sel de métal alcalin ou alcalino-terreux non toxique, de la précipitation des contaminants autres que le sel d'ammonium quaternaire par addition de 5 à 40% (volume/volume) d'un alcool miscible à l'eau suivie de l'élimination du sel d'ammonium quaternaire, caractérisé en ce qu'on élimine le sel d'ammonium quaternaire par précipitation par ajout d'un iodure, sulfocyanure ou benzoate soluble dans l'eau et qu'on sépare le précipité pour obtenir une solution de laquelle on isole ledit complexe.

2. Procédé suivant la revendication 1, dans laquelle la bactérie est Haemophilus influenzae type B.

3. Procédé suivant la revendication 1, dans laquelle la bactérie est Neisseria meningitidis groupe B.

4. Procédé suivant l'une quelconque des revendications 1 à 3, dans laquelle le milieu aqueux est un bouillon de culture.

5. Procédé suivant l'une quelconque des revendications 1 à 4, dans laquelle le sel d'ammonium quaternaire est le bromure de cétrimonium.

6. Procédé suivant l'une quelconque des revendications 1 à 5, dans laquelle le sel de métal non toxique est le chlorure de calcium.

7. Procédé suivant l'une quelconque des revendications 1 à 6, dans laquelle la quantité d'alcool miscible à l'eau est 25% (volume/volume) et l'alcool est l'éthanol.

8. Procédé suivant l'une quelconque des revendications 1 à 7 dans laquelle l'iodure soluble dans l'eau est un iodure de métal alcalin.

9. Procédé suivant l'une quelconque des revendications 1 à 8, dans laquelle l'isolement du complexe de la solution aqueuse est réalisé par ultrafiltration et lyophilisation.

## Patentansprüche für die Vertragsstaaten: BE, CH, DE, FR, GB, IT, LI, LU, NL, SE

1. Verfahren zur Herstellung eines immunogenen und lipopolysaccharidfreien, nicht-kovalenten Bakterienhüllen-polysaccharid-Protein-Komplexes, ausgehend von eingekapselten Bakterien, die in einem wäßrigen Medium suspendiert sind, wobei die Bakterien durch Zusatz eines quaternären Ammoniumsalzes inaktiviert, dann die unlösliche Fraktion abgetrennt und in einer 0,2 bis 2 N wäßrigen Lösung eines nicht toxischen Alkali- oder Erdalkalimetallsalzes aufgenommen, die außer dem quaternären Ammoniumsalz vorhandenen kontaminierenden Bestandteile durch Zusatz von 5 bis 40% (Volumen/Volumen) eines mit Wasser mischbaren Alkohols ausgefällt, und darauf das quaternäre Ammoniumsalz entfernt wird, dadurch gekennzeichnet, daß man das quaternäre Ammoniumsalz mittels Ausfällung durch Zusatz eines in Wasser löslichen Iodids, Rhodanids oder Benzoats entfernt, daß man den Niederschlag abtrennt und aus der so erhaltenen Lösung den Komplex isoliert.

2. Verfahren gemäß Anspruch 1, wobei das Bakterium Haemophilus influenzae Typ b ist.

3. Verfahren gemäß Anspruch 1, wobei das Bakterium Neisseria meningitidis Gruppe B ist.

4. Verfahren gemäß einem der Ansprüche 1 bis 3, wobei das wäßrige Medium eine Kulturbrühe ist.

5. Verfahren gemäß einem der Ansprüche 1 bis 4, wobei das quaternäre Ammoniumsalz Cetrimoniumbromid ist.

6. Verfahren gemäß einem der Ansprüche 1 bis 5, wobei das nicht toxische Metallsalz Calciumchlorid ist.

7. Verfahren gemäß einem der Ansprüche 1 bis 6, wobei die Menge des mit Wasser mischbaren Alkohols 25% (Volumen/Volumen) und der Alkohol Äthanol ist.

8. Verfahren gemäß einem der Ansprüche 1 bis 7, wobei das wasserlösliche Iodid ein Alkalimetalliodid ist.

9. Verfahren gemäß einem der Ansprüche 1 bis 8, wobei der Komplex aus der wäßrigen Lösung durch Ultrafiltration und Gefriertrocknung isoliert wird.

10. Immunogener, lipopolysaccharidfreier und nicht kovalenter Hüllenpolysaccharid-Protein-Komplex von Haemophilus influenzae Typ b, dadurch gekennzeichnet, daß die Polysaccharidfraktion aus Polyribosylribitolphosphat besteht und die Proteinfraktion einen Methionin-Rest pro Molekül aufweist und daß der Komplex bei Elektrophorese auf Polyacrylamid-Natriumdodecylsulfat-Gel zwei Flecke zeigt, die etwa 90% bzw. etwa 10% (Gewicht/Gewicht) Polypeptid entsprechen, das das im Komplex anwesende Protein darstellt, wobei die Hauptfraktion hydrophob ist und ein Molekulargewicht von etwa 41 000 Dalton aufweist und daß der Gesamtgehalt an Protein im Komplex mindestens 30% des Komplexes beträgt.

11. Komplex gemäß Anspruch 10, bei dem der Proteingehalt zwischen 40 und 90% (Gewicht/Gewicht) des Komplexes beträgt.

12. Impfstoff gegen Meningitis, dadurch gekennzeichnet, daß er eine wirksame Dosis des Polysaccharid-Protein-Komplexes gemäß einem der Ansprüche 10 und 11 in lyophilisierter Form mit einem Stabilisierungsmittel enthält.

**Patentansprüche für den Vertragsstaat AT**

1. Verfahren zur Herstellung eines immunogenen und lipolpolysaccharidfreien, nicht-kovalenten Bakterienhüllen-polysaccharid-Protein-Komplexes, ausgehend von eingekapselten Bakterien, die in einem wäßrigen Medium suspendiert sind, wobei die Bakterien durch Zusatz eines quanternären Ammoniumsalzes inaktiviert, dann die unlösliche Fraktion abgetrennt und in einer 0,2 bis 2 N wäßrigen Lösung eines nicht toxischen Alkali- oder Erdalkalimetallsalzes aufgenommen, die außer dem quaternären Ammoniumsalz vorhandenen kontaminierenden Bestandteile durch Zusatz von 5 bis 40% (Volumen/Volumen) eines mit Wasser mischbaren Alkohols ausgefällt, und darauf das quaternäre Ammoniumsalz entfernt wird, dadurch gekennzeichnet, daß man das quaternäre Ammoniumsalz mittels Ausfällung durch Zusatz eines in Wasser löslichen Iodids, Rhodanids oder Benzoats entfernt, daß man den Niederschlag abtrennt und aus der so erhaltenen Lösung den Komplex isoliert.

2. Verfahren gemäß Anspruch 1, wobei das Bakterium Haemophilus influenzae Typ b ist.

3. Verfahren gemäß Anspruch 1, wobei das Bakterium Neisseria meningitidis Gruppe B ist.

4. Verfahren gemäß einem der Ansprüche 1 bis 3, wobei das wäßrige Medium eine Kulturbrühe ist.

5. Verfahren gemäß einem der Ansprüche 1 bis 4, wobei das quanternäre Ammoniumsalz Cetrimoniumbromid ist.

6. Verfahren gemäß einem der Ansprüche 1 bis 5, wobei das nicht toxische Metallsalz Calciumchlorid ist.

7. Verfahren gemäß einem der Ansprüche 1 bis 6, wobei die Menge des mit Wasser mischbaren Alkohols 25% (Volumen/Volumen) und der Alkohol Äthanol ist.

8. Verfahren gemäß einem der Ansprüche 1 bis 7, wobei das wasserlösliche Iodid ein Alkalimetallodid ist.

9. Verfahren gemäß einem der Ansprüche 1 bis 8, wobei der Komplex aus der wäßrigen Lösung durch Ultrafiltration und Gefriertrocknung isoliert wird.

**Claims for the contracting states: BE, CH, DE, FR, GB, IT, LI, LU, NL, SE**

1. A process for preparing a non covalent, immunogenic lipopolysaccharide-free bacterial capsular polysaccharide-protein complex from encapsulated bacteria suspended in an aqueous medium which comprises inactivation of bacteria by the addition of a quaternary ammonium salt followed by collection of the insoluble fraction, take-up with a 0.2 to 2 N aqueous solution of a non toxic alkaline or alkaline-earth metal salt thereof, precipitation of contaminants other than the quaternary ammonium salt by addition of 5 to 40% (vol/vol) of a water-miscible alcohol followed by removal of the quaternary ammonium salt wherein the quaternary ammonium salt is removed by precipitation with a water soluble iodide, sulfocyanide or benzoate and the precipitate is separated to yield a solution from which said complex is isolated.

2. A process according to claim 1 wherein the bacteria is Haemophilus influenzae type b.

3. A process according to claim 1 wherein the bacteria is Neisseria meningitidis group B.

4. A process according to any of claims 1 to 3 wherein the aqueous medium is a culture broth.

5. A process according to any of claims 1 to 4 wherein the quaternary ammonium salt is cetrimonium bromide.

6. A process according to any of claim 1 to 5 wherein the non toxic metal salt is calcium chloride.

7. A process according to any of claims 1 to 6 wherein the amount of water-miscible alcohol is 25% (vol/vol) and the alcohol is ethanol.

8. A process according to any of claims 1 to 7 wherein the water-soluble iodide is an alkaline metal iodide.

9. A process according to any of claims 1 to 8 wherein the isolation of the complex from the aqueous solution is performed by ultrafiltration and lyophilisation.

10. A non covalent, immunogenic lipopolysaccharide-free capsular polysaccharide-protein complex of Haemophilus influenzae type b wherein the polysaccharide moiety is composed of polyribosylribitol phosphate and wherein the protein moiety presents one methionine residue per molecule and which, when tested by sodium dodecyl sulfate-polyacrylamide gel electrophoresis, shows two spots corresponding to about 90% and 10% (wt/wt) respectively of polypeptides accounting for the protein present in the complex, the main fraction being hydrophobic and of a molecular weight of about 41.000 daltons, the total amount of protein in the complex being at least 30% of the complex weight.

11. A complex according to claim 10 wherein the protein content is comprised between 40 and 90% (wt/wt) of the complex.

12. A meningitis vaccine comprising an effective dose of the polysaccharide-protein complex according to any of claims 10 and 11 in freeze-dryed form with a stabilizer.

**Claims for the contracting state: AT**

1. A process for preparing a non covalent, immunogenic lipopolysaccharide-free bacterial capsular polysaccharide-protein complex from encapsulated bacteria suspended in an aqueous medium which comprises inactivation of bacteria by the addition of a quaternary ammonium salt followed by collection of the insoluble fraction, take-up with a 0.2 to 2 N aqueous solution of a non toxic alkaline or alkaline-earth metal salt thereof, precipitation of contaminants other than the quaternary ammonium salt by addition of 5 to 40% (vol/vol) of a water-miscible alcohol followed by removal of the quaternary ammonium salt wherein the quaternary ammonium salt is removed by precipitation with a water soluble iodide, sulfocyanide or benzoate and the precipitate is separated to yield a solution from which said complex is isolated.

2. A process according to claim 1 wherein the bacteria is Haemophilus influenzae type b.

3. A process according to claim 1 wherein the bacteria is Neisseria meningitidis group B.

4. A process according to any of claims 1 to 3 wherein the aqueous medium is a culture broth.

5. A process according to any of claims 1 to 4 wherein the quaternary ammonium salt is cetrimonium bromide.

6. A process according to any of claims 1 to 5 wherein the non toxic metal salt is calcium chloride.

7. A process according to any of claims 1 to 6 wherein the amount of water-miscible alcohol is 25% (vol/vol) and the alcohol is ethanol.

8. A process according to any of claims 1 to 7 wherein the water-soluble iodide is an alkaline metal iodide.

9. A process according to any of claims 1 to 8 wherein the isolation of the complex from the aqueous solution is performed by ultrafiltration and lyophilisation.